Europäisches Patentamt

**European Patent Office**

**Office européen des brevets**

(11) Publication number: **0 306 915**

**A1**

(12) **EUROPEAN PATENT APPLICATION**

(21) Application number: **88114605.4**

(22) Date of filing: **07.09.88**

(51) Int. Cl.4: **A61B 5/03**

(30) Priority: **07.09.87 HU 398587**

(43) Date of publication of application:
**15.03.89 Bulletin 89/11**

(84) Designated Contracting States:
**AT DE GB**

(71) Applicant: **Mikroker Kisszövetkezet**
**Szondi u. 27**
**H-1067 Budapest(HU)**

(72) Inventor: **Török, Miklos, Dr.**
**Kmocsy u. 21**
**H-1141 Budapest(HU)**
Inventor: **Bartos, Imre**
**Ady E. u. 18**
**H-2040 Budaörs(HU)**

(74) Representative: **Lehn, Werner, Dipl.-Ing. et al**
**Hoffmann, Eitle & Partner Patentanwälte**
**Arabellastrasse 4**
**D-8000 München 81(DE)**

(54) Circuit arrangement for determining uterine contractions.

(57) The invention relates to a circuit arrangement for determining the number of uterine contractions, in particular for the prediction of the risk of premature labour, containing an analogue measuring unit, control logic with RAM, as well as at least one input device and a display. The output of the analogue measuring unit (1) is connected to the input of the contraction counter (5) via the uterine contraction detector (4). The output of the contraction counter (5) is connected to the data-lines of the RAM (3) and optionally to the input of the alarm unit (7). It is also connected to the display (9) (e.g. LCD) via the display control cct.(8). The address-inputs of the RAM (3) is connected to the outputs of the timing counter (6), while the control inputs of the RAM (3), the contraction counter (5) and the timing counter (6) are connected to the outputs of the control logic (2). The input of the control logic (2) is connected to an input unit (10), preferably keyboard or switch-array.

# CIRCUIT ARRANGEMENT FOR DETERMINING UTERINE CONTRACTIONS

The invention relates to a circuit arrangement for determining the number of uterine contractions, in particular for the prediction of the danger of premature birth; the circuit arrangement comprises an analogous measuring unit, a control logic and a data memory connected thereto, as well as at least one data-feeding organ and a display. On the basis of manifold tests it has become unambiguous that death of new-born children can be reduced by reducing the number of premature births; although the methods directed to the prevention of premature birth at our disposal, are considered, as efficient, their efficiency does not meet requirements from several aspects.

Screening of the women being increasingly endangered in respect to premature birth from the large number of pregnancies could not be solved up to now in a reliable way. In such a manner the majority of pregnant mothers subjected to hospital treatment spend several months unnecessary in hospitals, at the same time, women, whose premature birth starts later, do not come into the hospital in due time.

Preventive dispensation of medicaments which are most efficient for stopping uterine contractions is unacceptable in consideration of several side-effects for the mother and foetus. Adjustment of dosis becomes timely, when the pregnant mother has expressively contractions or regular contraction of the uterus. Adjustment of the doses of medicaments is based on the subjective complaints of the mother, i.e. whether she feels contractions or not.

Valve of operations directed to closing the orifice of the uterus in course of protective measures is questioned by a plurality of experts. If the orifice of the uterus is already open, an operation closing the orifice of uterus may be efficient, however, only in cases, if there is a real insufficiency of the cervix of the uterus and not the irregular, inactive, however, for a longer period active contractions of the "irritable" uterus form the background of happenings. In these cases the operation closing the orifice of the uterus may be even more harmful for the mother, as the basic problem is not at all solved, as more, more frequent and stronger contractions of the uterus may occur.

Non-invasive, permanent and ambulatory observation of uterine activity yields the promising possibility for avoiding the aforementioned difficulties. In such a manner complaints of the patient can be made objective and measurable.

It has been observed long ago, that by external permanent observation of uterine activity a part of the endangering premature births could be recognized early, prior to regular pains R. Bell; "The prediction of preterm labour by recording sponaneous antenatal uterine activity",British J. Obstet. Gynaeco. 1983. 90:884-887/

Contractions are also known, which are not efficient, and they can be considered as a normal uterine activity. /M. Katz, R.Roger et al.:"Assesment of uterine activity in ambulatory patients at high risk of premature labour and delivery"; Am. J. Obstet. Gynecol. 1986. 154: 44-47/.

Up to now two working teams elaborated a method for the permanent detection of uterine activity forming a part of the experimental programme "Home telemetry" known from professional literature /K. Dalton, J. Currie et. al.: "A computerized system for fetal home telemetry". Arch. Gynecol. 1985. 237: Supl. 33; R.P. Newman, P.G. Gill et al.: "Uterine activity during pregnancy in ambulatory patients; Comparison of singleton and twin gestations", Am. J. Obstet. Gynecol. 1986, 1954: 530-31/.

The apparatuses having been designed as part of the experimental programme are - as a matter of fact -the improved and portable versions of the monitors in the delivery-room, these are provided with an electronic memory for playing-back the data measured by telephone or to store them for the time of play-back.

The disadvantage of the apparatus lies in the relative large size in spite of portability, at the same time it is able to store the data of so about three hours, simultaneously evaluation of data requires daily play-back and continuous analysis of the obstetrician.

The aim of the invention is to develop a circuit arrangement, which is well suited for counting the number of uterine contractions, analysing the frequency and intensity of uterine contractions, as well as for storing the measured data for a longer period and/or optionally for local display.

A further requirement lies in the possibility of forwarding the data through a telephone-line and of the generation of alarm signal in case, a pre-set limit value is exceeded.

The invention is based on the recognition that a circuit arrangement meeting the requirements may be obtained, if the signals characterizing uterine contractions are measured and amplified, thereafter the signals are led to a counter counting contractions through a circuit observing the rhythm; the output of the contraction counter is connected to a data-memory and a display, optionally an alarming unit, while the function of the single units is coordinated by means of a control logic.

In accordance with the aim set the circuit arrangement according to the invention comprises for determining the number of contractions of the uterus, in particular for prediction of preterm labour, an analogous measuring unit, a control logic and a data memory connected thereto, as well as at least one data-feeding organ and a display and it is designed so, that the output of the analogous measuring unit is connected to the input of the contraction counter through a rhythm observing circuit, while the output of the contraction counter is connected partly to the data-lines of the data-memory and optionally to the input of the alarm unit, partly to the display through the control circuit, e.g. to an LCD display, furtheron, the address-inputs of the data-memory are connected to the outputs of the timing counter, while the input of the control logic is connected preferably to the data-feeding organ, preferably keyboard or a row of switches.

A further characteristic of the invention may lie in that the alarm unit is connected to a sensor sensing the limit value and the reference input thereof is connected to a limit value transmitter being suitable for determining the limit value, while the output is connected to some acoustic means, e.g. a miniature loudspeaker or a buzzer.

With a preferred embodiment of the invention the control logic comprises a basic oscillator and a timing unit connected to the output of the basic oscillator and controlled with the data-feeding organ.

With an other preferred embodiment the analogous measuring unit contains a sensor, a measuring amplifier connected to the input of the sensor and an analogous limit value sensor connected to the output of the measuring amplifier.

With another embodiment the analogous limit value sensor has two outputs, the first output is connected partly to the input of the rhythm observing circuit, partly to the input of the control circuit, while the other output is connected to a further input of the control circuit.

In any of the embodiments the circuit arrangement is completed with a data-interface and an interconnected modem, while the inputs of the data-interface are connected partly to the data-inputs of the data-memory and the address-inputs thereof, partly to the output of the control logic, at the same time the output of the modem is connected to the telephone-line.

The circuit arrangement according to the invention shows several advantageous features. Out of these the most advantageous characteristic lies in that -compared to known solutions - a far simpler and cheaper apparatus can be produced, which is well suited for the continuous observation of the condition of the pregnant woman in her own home, not requiring the presence of the doctor.

It is considered as advantageous, in so far as the apparatus is well suitable for storing measuring data for more than 24 hours and in case of necessity for the display of the data stored.

In case of a dangerous uterine activity a warning signal can be generated, after said signal the pregnant woman may avail herself of medical care.

Furtheron, it is considered as advantageous, that by virtue of the simple design the number of required components is low, consequently layout in a small size becomes possible, so wearing does not involve particular discomfort for the pregnant mother.

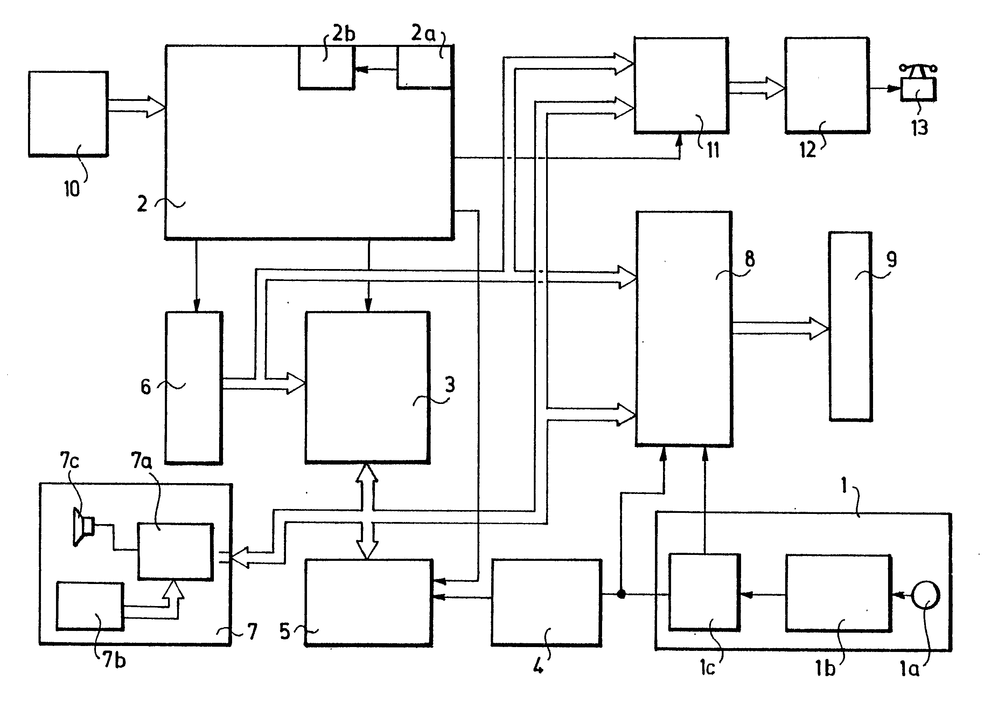

The invention will be described in detail by the aid of a preferred embodiment with reference to the drawing enclosed, illustrating the detailed block-scheme of a possible circuit arrangement.

The most important components of the circuit arrangement are the analogous measuring unit 1, the control logic 2, the data-memory 3, the rhythm observing circuit 4, the contraction counter 5, the timing counter 6, as well as the display 9.

The analogous measuring unit 1 is provided with a sensor known in itself, which may be a highsensitive temperature-compensated remote-transmitter transmitting pressure activated piezoelectrically or provided with a strain gauge.

Sensor 1a is connected to the analogous limit value sensor 1c via the measuring-amplifier 1b. Analogous limit value sensor 1b has two outputs, one output is connected to the input of the rhythm observing circuit 4 and to the input of the displaying control circuit 8, while the other output of the analogous limit value sensor 1c is connected to the other inputs of the displaying control circuit 8, the output of which is connected to the inputs of the display 9, e.g. an LCD display.

A preferred embodiment of the rhythm observing circuit 4 may include a resetable monostable multivibrator, a differentiating member connected thereto and a NAND-gate interconnecting the output of the differentiating member and the input of the monostable multivibrator. Input of the rhythm observing circuit 4 is formed by the input of the monostable multivibrator, while the output is formed by the output of the NAND-gate.

The output of the rhythm observing circuit 4 is connected to the input of the contraction counter 5, while the output thereof is connected to the data-input of the data-memory 3, furtheron, to the inputs of the alarm unit 7, the displaying control circuit 8, as well as to the data-interface 11.

As a data-memory 3 e.g. 64 x 4 bit RAM may be used, however, a memory with a larger storing capacity can also be used.

The main component of the alarm unit 7 is the limit value sensor 7a, the reference input of which is connected to the limit value transmitter 7b, while the output is connected with the acoustic device 7c. Preferably, as a limit value sensor 7a a digital comparator is used, while a miniature loudspeaker serves as acoustic device 7c.

The control logic 2 is provided with the basic oscillator 2a, which is connected to the timing unit 2b. The input of the control logic 2 is connected to the data-feeding organ 10, being expediently a keyboard or a row of switches.

With a preferred embodiment the basic oscillator 2a of the control logic 2 is a low-frequency RC oscillator, the output of which is connected to an eight-bit synchron counter forming the timing unit 2b.

The outputs of the control logic 2 are connected to the control inputs of the data-memory 3, the timing counter 6, the contraction counter 5 and the data-interface 11.

The outputs of the timing counter 6 are connected partly to the address-inputs of the data-memory 3, of the displaying control circuit 8, partly of the data-interface 11.

The output of the data-interface 11 is connected to the modem 12, while the output thereof is connected to the telephone-line 13. The modem 12 comprises expediently a microprocessor-controlled parallel/to serial converter and a standardized modem.

The circuit arrangement according to the invention operates as follows;

Timing signals needed for operation are generated by the timing unit 2b of the control logic 2 from the signal of the basic oscillator 2a. The task of the control logic 2 lies in partly to generate the basic clock signal of the contraction counter 5, partly to control the apparatus in dependence of the adjustment of the row of switches forming the data-feeding organ 10.

With the embodiment described here - serving as an example - the task of the rhythm observing circuit 4 is solved so, in so far as the transition 0-1 of the signal coming from the analogous limit value sensor 1c set free the reset input of the monostable multivibrator thus generating the starting pulse.

At the end of timing or in case of inhibition on the output of the differentiating member - connected to the output of the monostable multivibrator - a positive pulse will be generated.

Thereafter, from the input signal and the output signal of the differentiating member the NAND-gate generates the output signal of the rhythm observing circuit 4.

Uterine activity of the pregnant mother is detected by the sensor 1a, while the detected signal is amplified in the measuring-amplifier 1b.

The amplified signal of the measuring-amplifier 1b is processed in the analogous limit value sensor 1c, thereafter the signal is forwarded partly to the rhythm observing circuit 4, partly to the displaying control circuit 8.

The task of the rhythm observing circuit 4 lies partly in to decide, whether the content of the signal coming from the analogous limit value sensor 1c corresponds to an uterine contraction, partly to convert the analoguous signal into a digital signal and in case of a signal being characteristic for the contraction of the uterine to forward it to the contraction counter 5.

Contractions of the uterine are counted for a pre-set time, e.g. for an hour. After the expiration of the pre-set period using the output signal of the eight-bit counter serving as the timing unit 2b is loaded to the address of the date-memory 3 having been defined by the timing counter 6, thereafter the contraction counter 5 is reset and the timing counter 6 is allowed to shift forward.

Counting time and actual value of the contraction counter 5 can be read on the display 9.

Momentary signal of the contraction counter 5 is led to the alarm unit 7 too. The alarm unit 7 observes actual values to the contraction counter 5 and in case, if said value exceeds the value having been re-set on the limit value transmitter 7b, an alarm signal will be generated with the acoustic means 7c.

When using the apparatus, it is placed onto the belly of the pregnant mother, the strap of the apparatus is stretched to the extent, that limit value of measuring could be achieved. Now, on the first output of the analogous limit value sensor 1c the active logical level appears, which is displayed by the change of e.g. the right extreme digit of the LCD display, forming the display 9 from 0 to 8.

When reading back to the data contained in the data memory 3, these can be displayed by means of the data-feeding organ 10 on the display 9 so, that the sixteen-bit counter is set into the basic position and by pushing the button of the data-feeding organ 10 the timing counter 6 is put into action, meanwhile the data of the data-memory 3 may be read on the display 9. It is also possible to feed the stored data to the computer of the physician through the data-interface 11 and modem 12, via the telephone-line 13.

In this case conditions of the timing counter 6, the contraction counter 5 and the data-feeding organ 10 are stored provisionally in the data-interface 11, thereafter the data are forwarded to the modem 12.

It is also possible to allow the apparatus to operate periodicallly, in this case the data-memory 3, the contraction counter 5 and the timing counter 6 remain in their connected state, while the other circuits are disconnected from the supply voltage source, accordingly an useful life thereof can be prolonged.

The apparatus according to the invention can be expediently used for the continuous observation of pregnant mothers in their own home in case of risk of premature birth, consequently, for eliminating dangerous consequences of preterm labour.

## Claims

1. Circuit arrangements for determining the number of uterine contraction, in particular for the prediction of the risk of premature labour, containing an analogous measuring unit, a control logic and a data-memory connected thereto, as well as at least one data-feeding organ and a display, **characterized** in that the output of the analogous measuring unit (1) is connected to the input of the contraction counter (5) through the rhythm observing circuit (4), the output of the contraction counter (5) is connected partly to the data-lines of the data-memory (3) and optionally to the input of the alarming unit (7), partly through the displaying control circuit (8) to the display (9), e.g. an LCD display, furtheron, the address-inputs of the data-memory (3) are connected to the outputs of the timing counter (6), while the control inputs of the data-memory (3), the contraction counter (5) and the timing counter (6) are connected to the outputs of the control logic (2), and the inputs of the control logic (2) are connected expediently to the data-feeding unit (10), preferably keyboard or switching organ.

2. Circuit arrangement as claimed in claim 1, **characterized** in that the alarm unit (7) is provided with a limit value sensor (7a), the reference input of which is connected to a limit value transmitter (7b) being suitable for determining the limit value, and the output is connected to acoustic means (7c), e.g. a miniature loud-speaker.

3. Circuit arrangement as claimed in claim 1 or 2, **characterized** in that as control logic (2) a basic oscillator (2a) is used and said oscillator comprises a timing unit (2b) connected to the output thereof and controlled with a data-feeding unit (10).

4. Circuit arrangement as claimed in any of the claims 1 to 3, **characterized** in that the analogous measuring unit (1) comprises a sensor (1a), a measuring amplifier (1b) connected to the input of the sensor (1a), as well as an analogous limit value sensor (1c) connected to the output of the measuring amplifier (1b).

5. Circuit arrangement as claimed in claim 4, **characterized** in that the analogous limit value sensor (1c) has two outputs, the first output is connected partly to the input of the rhythm observing circuit (4), partly to the input of the displaying control circuit (8), while the second output is connected to the further input of the displaying control circuit (8).

6. Circuit arrangement as claimed in any of the claims 1 to 5, **characterized** in that the circuit is completed with a data-interface (11) and the inputs thereof are connected partly to the data-inputs and address inputs of the data-memory (3), partly to the outputs of the control logic (2), while the outputs of the data-interface (11) are connected to the telephone-line (13) via a modem (12).

EP 0 306 915 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | WO-A-8 603 115 (J.G.CLARE) * Abstract; page 1, line 9 - page 2, line 8; page 7, line 32 - page 9, line 1; figures 8,9 * | 1-4 | A 61 B 5/03 |
| A | | 5,6 | |
| | --- | | |
| Y | US-E- 31 158 (M.C.CARTER et al.) * Abstract; column 3, line 63 - column 4, line 64; figure 1 * | 1-4 | |
| A | | 5,6 | |
| | --- | | |
| A | PROCEEDINGS OF THE IEEE, vol. 65, no. 5, May 1977, pages 722-729, New York, US; W.C.LIN et al.: "A microprocessor-based data acquisition and processing system for studying the kinematics of labor" * Abstract; page 723, paragraph II: "Data Acquisition System"; figures 1-4 * | 1-6 | |
| | --- | | TECHNICAL FIELDS SEARCHED (Int. Cl.4) |
| A | FR-A-2 403 058 (A.DITTMAR et al.) * Page 3, line 39 - page 4, line 40; figures 1,2 * | 1-6 | A 61 B |
| | --- | | |
| A | US-A-4 608 994 (L.OZAWA et al.) * Abstract; figures 1,2,4 * | 6 | |
| | ----- | | |

The present search report has been drawn up for all claims

| Place of search | Date of completion of the search | Examiner |
|---|---|---|
| THE HAGUE | 05-12-1988 | HUNT,B.W. |

EPO FORM 1503 03.82 (P0401)